(19) Europäisches Patentamt
European Patent Office
Office européen des brevets

(11) Publication number : **0 420 552 B1**

(12) # EUROPEAN PATENT SPECIFICATION

(45) Date of publication of patent specification : **15.06.94 Bulletin 94/24**

(51) Int. Cl.$^5$ : **C07H 15/244,** A61K 31/71, C12P 19/56, // (C12P19/56, C12R1:04)

(21) Application number : **90310433.9**

(22) Date of filing : **24.09.90**

(54) **New antifungal antibiotic, and the production and uses of same.**

(30) Priority : **26.09.89 JP 248143/89**

(43) Date of publication of application : **03.04.91 Bulletin 91/14**

(45) Publication of the grant of the patent : **15.06.94 Bulletin 94/24**

(84) Designated Contracting States : **DE FR GB IT**

(56) References cited : **EP-A- 0 315 147**

(73) Proprietor : **ZAIDAN HOJIN BISEIBUTSU KAGAKU KENKYU KAI**
**14-23, Kami Ohsaki 3-chome**
**Shinagawa-ku Tokyo (JP)**

(72) Inventor : **Takeuchi, Tomio**
**New Fuji Mansion 701,**
**1-11,Higashi Gotanda 5-Chome**
**Shinagawa-ku, Tokyo (JP)**
Inventor : **Hamada, Masa**
**Shuwa Residence 405,**
**1-26, Naito-machi**
**Shinjuku-ku, Tokyo (JP)**
Inventor : **Kondo, Shinichi**
**1157-1-801, Ichigao-cho,**
**Midori-ku**
**Yokohama-shi, Kanagawa-ken (JP)**
Inventor : **Sezaki, Masaji**
**12-16 Sangenjaya 1-Chome,**
**Setagaya-ku**
**Tokyo (JP)**
Inventor : **Gomi, Shuichi**
**17-17-202 Kamiikedai 3-Chome,**
**Ohta-ku**
**Tokyo (JP)**

(74) Representative : **Baverstock, Michael George Douglas et al**
**BOULT, WADE & TENNANT**
**27 Furnival Street**
**London, EC4A 1PQ (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 420 552 B1

Jouve, 18, rue Saint-Denis, 75001 PARIS

## Description

This invention relates to a new antifungal antibiotic, dexylosylbenanomicin A and salts thereof which are useful as a therapeutic antifungal agents. This invention also relates to a pharmaceutical composition comprising dexylosylbenanomicin A or a salt thereof as active ingredient. This invention further relates to processes for the production of dexylosylbenanomicin A.

Many antibiotics are already known, but new antibiotic substances are still in demand as pharmaceutical agents. Compounds which are already known and are similar in their molecular structural features to the new antifungal antibiotic, dexylosylbenanomicin A and salts thereof now newly provided by the present inventors include benanomicin A, benanomicin B and dexylosylbenanomicin B (see Japanese patent applications Nos. 277,692/87 and 327,163/87 as laid-open under Japanese patent application publications "Kokai" No. 121293/83 published on 12 May, 1989 and "Kokai" No. 168,694/89 published on 4 July 1989, respectively, as well as the corresponding European patent application publication No. 0 315 147 A2 published on 10 May 1989 and corresponding U.S. patent application SN. 264,888 filed on 31 October 1988) as well as pradimicins A, B and C [Oki et al: "Journal of Antibiotics" 41, 1701-1704 (1988); Tsunakawa et al: "J. Org. Chem." 54, 2532-2536 (1989)].

Hitherto, a variety of antibiotics which are produced by microorganisms are already known. Among the known antibiotics, however, such antifungal antibiotics which can exhibit excellent antifungal effects but a low toxicity to mammals are only few. Accordingly, there is a high demand for a new antifungal antibiotic which is useful in the therapeutic treatment of various fungal infections in mammalian animals, including humans.

According to inventions made earlier by the present inventors and their associates, there are provided two antibiotics, benanomicin A and benanomicin B, which each have an antifungal activity and an HIV-inactivating activity, as well as a process for the production of benanomicins A and B by cultivation of an actinomycete strain MH193-16F4 (see the above-mentioned European patent application publication No. 0 315 147 A2 and its corresponding U.S. patent applicaiton SN. 264,888). The chemical structure of benanomicin A is shown by the following formula (II)

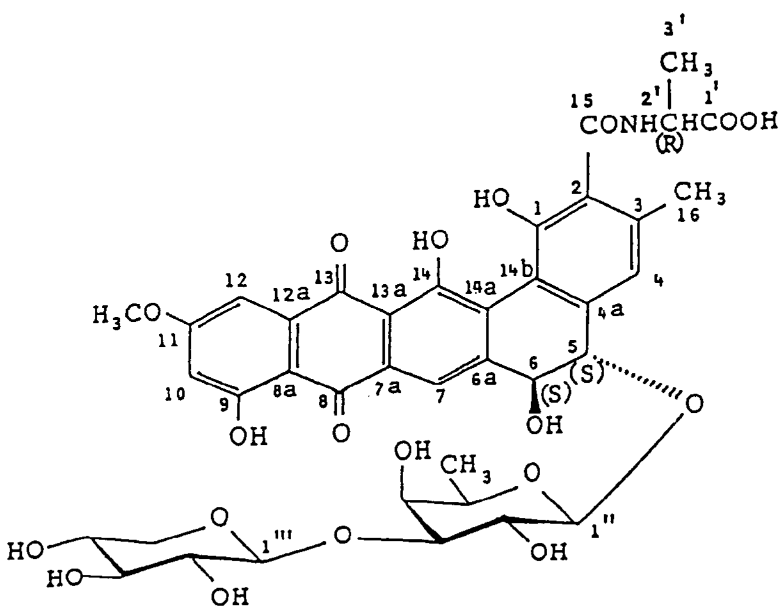

(II)

As derivatives of benanomicins A and B, there are also earlier provided dexylsoylbenanomicin B (said U.S. patent application SN. 264,888) and N-acetylbenanomicin B (Japanese patent application No. 4701/89, filed on January 13, 1989 and its corresponding U.S. patent application SN. 459,352 and corresponding European patent application publicaiton No. 0 378 126).

The present inventors have continued their researches with a view to providing new derivatives of benanomicin A which show a usefully high antifungal activity and are useful as therapeutic antifungal agents for the treatment of fungal infections in mammals. As a result, the inventors have now suceeded in chemically synthetizing a new compound, dexylosylbenanomicin A from benanomicin A and have found that dexylosylbenanomicin A exhibits antifungal activity.

An object of this invention is to provide a new antifungal antibiotic, dexylosylbenanomicin A and salts

thereof which exhibit usefully high antifungal activity. Another object of this invention is to provide processes for the production of the new antifungal antibiotic, dexylosylbenanomicin A. Further objects of this invention will be clear from the following description.

In a first aspect of this invention there is provided a new antifungal antibiotic, dexylosylbenanomicin A having the following formula (I)

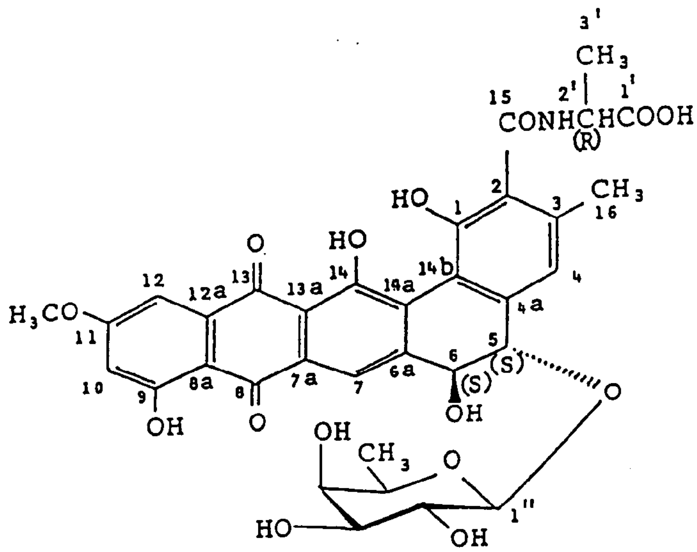

(I)

and salts thereof.

The salts of dexylosylbenanomicin A include pharmaceutically acceptable salts for example, pharmaceutically acceptable alkali metal salt such as potassium, sodium and lithium salts (the carboxylates) at the carboxyl group at the 1′-position of dexylosylbenanomicin A, and the ammonium salt, as well as pharmaceutically acceptable alkaline earth metal salts, such as calcium and magnesium salts (the carboxylates) at the said carboxyl group of dexylosybenanomicin A. Salts of dexylosylbenanomicin A further include pharmaceutically acceptable base-addition salts (at the carboxyl group of dexylosylbenanomicin A) with pharmaceutically acceptable organic bases, particularly amines, such as a lower ($C_1$ - $C_6$) alkyl amines, especially triethylamine, ethanolamine and dicyclohexylamine.

Dexylosylbenanomicin A according to this invention has the following characteristics:-

1. Physicochemical properties of dexylosylbenanomicin A are listed below.

(1) Color and appearance: Dark reddish powder

(2) Empirical formula: $C_{34}H_{33}NO_{15}$

(3) Mass spectrometry (FD-MS): m/z 696$(M+1)^+$

(4) Melting point: >200°C

(5) Ultraviolet and visible-ray absorption spectrum,

$\lambda$max, nm ($E^{1\%}_{1\,cm}$ ):

| | |
|---|---|
| [In methanol]: | 209(610), 229(595), 286(480), 300sh(385), 400sh(120), 479(198) |
| [In 0.1 N HCl-methanol]: | 211(578), 234(643), 298(577), 400sh(150), 458(225), |
| [In 0.1 N NaOH-methanol]: | 215(1269), 248(672), 260sh(590), 319(312), 496(294) |

(6) Infrared absorption spectrum (KBr, $cm^{-1}$):

3370, 2970, 2910, 1720, 1620, 1600, 1510, 1490, 1450, 1430, 1390, 1380, 1340, 1300, 1260, 1240, 1210, 1165, 1150, 1140, 1075, 1040, 1000, 970, 900, 875, 835, 810, 750

(7) $^1H$-NMR spectrum (400 MHz, in DMSO-$d_6$, 40°C)

δ(ppm): 1.13(3H, d), 1.34(3H, d), 2.32(3H, s), 3.38(1H, dd), 3.44(1H, br d), 3.52(1H, br), 3.56(1H br q), 3.95(3H, s), 4.42(1H. dq), 4.47(1H, br d), 4.54(1H, br d), 4.54(1H, d), 6.92(1H, d), 7.18(1H, br s), 7.30(1H, d), 8.06(1H, br s), 8.49(1H, d), 12.46(1H, br), 12.87(1H, s)

(8) $^{13}C$-NMR spectrum (100 MHz, in DMSO-$d_6$, 40°C)

δ(ppm): 187.4 s, 184.9 s, 173.9 s, 166.9 s, 165.9 s, 164.7 s, 156.8 s, 151.0 s, 147,9 s, 138.2 s, 137.3 s, 134.2 s, 131.3 s, 127.4 s, 125.6 s, 118.5 d, 115.4 d, 115.4 s, 113.6 s, 110.0 s, 107.6 d, 106.8 d, 105.2 d, 81.7 d, 73.5 d, 71.9 d, 71.2 d, 71.0 d, 70.3 d, 56.3 q. 47.6 d, 19.1 q, 16.8 q, 16.5 q

(9) Solubility: Sparingly soluble in chloroform, ethyl acetate, acetone and methanol but soluble in dimethyl sulfoxide and N,N-dimethylformamide.

(10) Distinction between the basic, acidic and neutral natures of substance: Acidic substance.

With reference to the accompanying drawings:-

Figure 1 shows ultraviolet and visible-ray absorption spectra of dexylosylbenanomicin A in methanolic solutions (20 μg/ml), wherein the spectrum of dexylosylbenanomicin A as measured in methanol is shown by a full line; the spectrum of dexylosylbenanomicin A as measured in 0.1 N hydrogen chloride-methanol is shown by a broken line; and that as measured in 0.1 N sodium hydroxidemethanol is shown by a chain line.

Figure 2 shows an infrared absorption spectrum of dexylosylbenanomicin A as pelleted in potassium bromide.

Figure 3 shows a $^{1}H$-NMR absorption spectrum of dexylosylbenanomicin A as measured at 400 MHz in deuterodimethyl sulfoxide (DMSO-$d_6$).

Figure 4 shows a $^{13}C$-NMR absorption spectrum of dexylosylbenanomicin A as measured at 100 MHz in deuterodimethyl sulfoxide (DMSO-$d_6$)

2. Antifungal activity of dexylosylbenanomicin A is now described.

The minimum inhibitory concentrations (MIC., mcg/ml) of dexylosylbenanomicin A against a variety of fungi were determined by a standard serial dilution method on a nutrient agar medium containing 1% glucose (pH 7.0) after incubation at 27°C for 42 hours and are shown in Table 1 below.

Table 1

| Microorganism tested (fungi) | Minimum inhibitory concentrations (μg/ml) |
|---|---|
| Candida tropicalis F-1 | 12.5 |
| Candida pseudotropicalis F-2 | 3.13 |
| Candida albicans 3147 | 12.5 |
| Candida Yu-1200 | 6.25 |
| Candida krusei F-5 | 50 |
| Saccharomyces cerivisiae F-7 | 3.13 |
| Cryptococcus neoformans F-10 | 12.5 |
| Cochliobolus miyabeanus | >25 |
| Pyricularia oryzae | 50 |
| Pellicularia sasakii | 25 |
| Xanthomonas citri | >100 |
| Xanthomonas oryzae | 100 |
| Aspergillus niger F-16 | 50 |
| Trichophyton asteroides 429 | >25 |
| Trichophyton mentagrophytes (883) | >25 |

Dexylosylbenanomicin A is of low toxicity to mammals, as it was shown that for estimation of acute toxicity in mice upon intravenous administration, dexylosylbenanomicin A was administered via the intravenous route to ICR mice (female, 4-week old, body weight of 19 to 21 g), the mice tolerated a dosage of 300 mg/kg of this new antibiotic.

In a second aspect of this invention, there is provided an antifungal composition for therapeutic treatment of a fungal infection in mammalian animals, including humans, which comprises an antifungally effective amount of dexylosylbenanomicin A having the formula (I) as defined hereinbefore or a pharmaceutically acceptable salt thereof, as active ingredient, in association with a pharmaceutically acceptable solid or liquid carrier.

The pharmaceutical antifungal composition containing the new compound of the formula (I) according to this invention as the active ingredient may be formulated in a known manner into a conventional formulation for administration, for example, powder, granules, tablets, pills and capsules for oral administration, as well as intravenously, intramuscularly or subcutaneously injectable solution, and suppositories, using a pharmaceutically acceptable solid or liquid carrier which is suitable for the intended formulation.

In general, the new compound of the formula (I) according to this invention can be administered either orally or parenterally upon its actual administration in the form of an antifungal composition.

When the active ingredient compound of the formula (I) according to this invention is given as an antifungal agent, it can be administered alone or it can be administered in the form of an injection, oral preparation, suppository or the like containing an excipient or carrier mixed together with the compound. Any pharmaceutically acceptable excipient and carrier may be used for that purpose. The nature and quantity of the carrier used may vary depending on the administration route and manner. For example, water, ethanol, an animal or vegetable oil such as soybean oil, sesame oil or mineral oil, or a synthetic oil may be used as a liquid carrier. Suitable solid carriers include, for example, a sugar such as maltose or sucrose, an amino acid, a cellulose derivative such as hydroxypropylcellulose, an oligosaccharide such as cyclodextrin, a salt of an organic acid such as magnesium stearate, or the like. In the case of the injections, it is generally preferable that the liquid medium of the injections comprises physiological saline, a buffered solution, an aqueous solution of a sugar such as glucose, inositol or mannitol, or a glycol such as ethylene glycol or polyethylene glycol. It is also feasible to formulate a lyophilized preparation containing the benanomicin A derivative of the formula (I) as the active ingredient mixed with an excipient, e.g., a sugar such as inositol, mannitol, glucose, mannose, maltose or sucrose or an amino acid such as phenylalanine. Upon administration such a lyophilized preparation may be dissolved in a suitable solvent for injection, for example, sterilized water or an intravenously-administerable liquid such as physiological saline, aqueous solution of glucose, an aqueous solution of electrolytes or an aqueous solution of amino acid.

Although the proportion of the benanomicin A derivative of the formula (I) present in the formulated composition may vary widely from one preparation to another, it may generally be in a range of 0.1-100% by weight. In the case of an injection, for example, it is generally desirable that the injectable solution contains the compound of the formula (I) as active ingredient at a concentration of 0.1-20% by weight. For oral administration, the compound of the formula (I) may be formulated into tablets, capsules, a powder or granules in mixture with a solid carrier or it may also be formulated into a solution, a dry syrup or the like in combination with a liquid carrier. In capsules, tablets, granules or a powder, the proportion of the benanomicin A derivative of the formula (I) present therein may generally be in a range of about 3-100%, preferably 10-100% by weight, with the balance being a carrier or carriers.

The dosage of the benanomicin A derivative of the formula (I) may suitably be determined taking into account the age, body weight and symptoms of patients and the therapeutic purpose intended. The therapeutic, i.e., effective dosage of the benanomicin A derivative of the formula (I) may be generally in a range of 1-50 mg/kg/day for parenteral administration and in a range of 5-100 mg/kg/day for the oral administration. This dosage can be administered either continuously or intermittently as long as the total dosage does not exceed the maximum level indicated to be prudent in view of the results of animal tests and various circumstances. Similarly, the total dosage given by parenteral administration may, of course, vary suitably depending on the method of administration, the condition of the patient or animal under treatment, for example, the age, body weight, sex, sensitivity, foods or feed, administration time, administration route, drugs administered concurrently, condition of the patient, and the disease. The suitable dosage and administration frequency of the benanomicin A derivative of the formula (I) under given conditions must be determined by an expert physician through tests to determine optimal dosage and in the light of the above guidelines. These requirements for administration also apply to the oral administration of benanomicin A derivatives of formula (I) according to this invention.

A third aspect of this invention provides a process for the production of an antifungal antibiotic, dexylosylbenanomicin A having the formula (I) as defined hereinbefore, which comprises converting chemically the known antifungal antibiotic, benanomicin A to give dexylosylbenanomicin A.

The method for preparing the starting material, benanomicin A having the formula (II) shown hereinbefore is described in the specification of the aforesaid European patent application publication No. 0 315 147 A2, but the preparation of benanomicin A is briefly illustrated hereinafter with reference to Reference Examples 1 to 2.

Briefly, the fermentative production of the antibiotic, benanomicin A may be carried out by inoculating the MH193-16F4 strain of actinomycete (identified as a microorganism deposited under "FERM BP-2051" in the Japanese depository "Fermentation Research Institute" at Tsukuba City, Japan since August 21, 1987) to a culture medium containing such nutrient sources as can be utilized by ordinary microorganisms, and then incubating said benanomicin-producing strain under aerobic conditions. Benanomicin A is produced together with benanomicin B and they are accumulated primarily in the culture broth. Benanomicins A and B may be recovered from the resulting culture, especially from the culture broth or its filtrate and then be separated from each other.

In the process for the production of dexylosylbenanomicin A according to the third aspect of this invention,

the reactions for chemical conversion of the starting benanomicin A into dexylosylbenanomicin A may comprise oxidizing benanomicin A by treatment with periodic acid or a metal periodate, and then reducing the resulting product of oxidation of benanomicin A by treatment with a reducing agent such as a hydride.

More particularly, the process for the production of dexylosylbenanomicin A from benanomicin A may be conducted by reacting benanomicin A with periodic acid or an alkali metal periodate, for example, sodium methaperiodate and potassium methaperiodate in solution in water at room temperature, so that the adjacent hydroxyl groups of the xylosyl moiety of benanomicin A are oxidized to give a diformyl compound as derived from benanomicin A. Said diformyl compound derived from benanomicin A is a compound which contains two formyl groups and has an empirical formula $C_{38}H_{37}NO_{18}$. This diformyl derivative compound is then treated with a reducing agent, for example, a reducing hydride such as sodium borohydride, boron sodium cyanohydride and the like, to produce dexylosylbenanomicin A having the formula (I) in the resulting reaction solution. We have now found that dexylosylbenanomicin A can successfully be produced from benanomicin A through the above-mentioned reaction steps comprising the periodate oxidation of benanomicin A, followed by reductive cleavage of the oxidized derivative of benanomicin A, contrary to the experimental fact that dexylosylbenanomicin A cannot be produced so far by direct hydrolysis or alcoholysis of benanomicin A.

For recovery of dexylosylbenanomicin A from the reaction solution containing it, dexylosylbenanomicin A can be separated from the reaction solution and then purified by using conventional methods for recovery and purification of chemical substances, for example, solvent extraction, the ion-exchange resin method, adsorptive or partition column chromatography, gel filtration, dialysis, precipitation and the like, either singly or in combination. For instance, dexylosylbenanomicin A present in the aqueous reaction solution can be adsorbed by a macroreticular resin commercially available under a tradename "Diaion HP-20" (a product from Mitsubishi Kasei Co., Ltd., Japan). For purification of dexylosylbenanomicin A, it is possible to utilize a charomatographic method with silica gel (commercially available under a tradename "WAKOGEL C-300", a product from Wako Pure Chemical Industries Ltd., Japan), alumina or other suitable adsorbents or with a gel-filtration agent such as "Sephadex LH-20" (a tradename of a product from Pharmacia AB, Sweden).

Dexylosylbenanomicin A which has been formed and is present in the above-mentioned reaction solution can be separated in its free form, that is, in the form of dexylosylbenanomicin A itself from the reaction solution. Additionally, when a solution or a concentrated solution containing dexylosylbenanomicin A is treated during the operations of the different stages for purification of dexylosylbenanomicin A with a base, for example, an inorganic base such as an alkali metal compound, e.g., sodium hydroxide, potassium hydroxide and the like, or ammonium hydroxide, as well as an alkaline earth metal compound, e.g. calcium hydroxide, magnesium hydroxide and the like, or an organic base such as an alkylamine, e.g. ethanolamine, triethylamine, dicyclohexylamine and the like, dexylosylbenanomicin A can be converted into its corresponding salts more particularly the metal salt (the carboxylates) or base addition salts at the carboxyl group of dexylosylbenanomicin A, which can then be separated from the reaction solution. On the other hand, salts of dexylosylbenanomicin A produced as above can be converted again into the free form, namely dexylosylbenanomicin A itself by a conventional methods such as treatment with mineral acids. Further, dexylosylbenanomicin A obtained in its free form can be converted into its salt forms by treatment with the above-mentioned bases in a known manner.

Accordingly, dexylosylbenanomicin A as well as its salts as mentioned above are embraced within the scope of this invention.

Furthermore, it has recently been found that when the above-mentioned MH193-16F4 strain of actinomycetes (identified as the microbial strain deposited under the access number "FERM BP-2051") is cultivated in a liquid culture medium for a sufficient time on a large scale, the resulting culture broth contains a substantial amount of dexylosylbenanomicin A is produced and accumulated along with benanomicins A and B, and that this dexylosylbenanomicin A can be recovered from the culture broth of the MH193-16F4 strain. In an additional aspect of this invention, therefore, there is provided a process for the fermentative production of dexylosylbenanomicin A, which comprises cultivating an MH193-16F4 strain of actinomycetes (identified as "FERM BP-2051") in a culture medium containing nutritional and assimilable carbon and nitrogen sources under aerobic conditions until a substantial amount of dexylosylbenanomicin A is produced and accumulated in the culture along with benanomicins A and B, and then recovering dexylosylbenanomicin A from the culture.

In the process for the fermentative production of dexylosylbenanomicin A, the cultivation of the MH193-16F4 strain may be done in the same manner as in the fermentative production of benanomicins A and B as described in the aforesaid European patent application publication No. 0 315 147. The culture broth filtrate obtained and containing benanomicins A and B as well as dexylosylbenanomicin A is treated with a macroreticular resin such as "Diaion HP-20" for adsorption of these antibiotic substances, followed by washing with water and eluting the adsorbent resin having adsorbed the antibiotic substances with aqueous methanol to give fractions of the eluate separately from the fractions containing benanomicin B. The active fractions of

eluate which contain benanomicin A together with dexylosylbenanomicin A are combined and concentrated in vacuo and the resultant concentrated solution is then acidified to an acidic pH by addition of dilute hydrochloric acid, to obtain a precipitate comprising benanomicin A and dexylosylbenanomicin A in admixture. This precipitate is collected and dried in vacuo and dissolved in N,N-dimethylformamide, and the resulting solution is saturated with water vapor at room remperature for 3 days until a crystalline precipitate mainly comprising benanomicin A-dimethylformamide solvate is deposited. The crystalline precipitate is collected by filtration and the resulting filtrate (the mother liquor) is concentrated to dryness in vacuo to give a crude powder. This crude powder is then dissolved in an aqueous alkaline solution containing sodium hydroxide (pH 9.0) and the solution is acidified to a pH of about 3.0 by addition of hydrochloric acid to deposit a brownish red precipitate. When this reddish brown solid powder is then purified by chromatography on a reverse-phase column "Cosmosil $75C_{18}$-OPN" (Nacalai Tesque, Inc., Japan) developed with aqueous methanol, dexylosylbenanomicin A in the form of a reddish brown powder can be isolated.

Moreover, this invention embraces a use of dexylosylbenanomicin A or a pharmaceutically acceptable salt thereof in the manufacture of an antifungal agent, especially a therapeutic antifungal drug.

This invention is now illustrated with reference to the following Examples, to which this invention is not limited in any way. Thus, the detailed properties of dexylosylbenanomicin A have been made evident by this invention and hence it is feasible for those skilled in the art to understand and perform the processes of producing dexylosylbenanomicin A in different ways while taking into account the above-described properties of this compound. Accordingly, this invention embraces not only any modification of the procedures of the following Examples, but also all such processes wherein dexylosylbenanomicin A is produced, concentrated, extracted and/or purified in a manner known per se whilst utilizing the properties of dexylosylbenanomicin A.

The following Reference Examples 1 to 2 illustrate the fermentative production of benanomicin A.

Reference Example 1

A loopful quantity of the MH193-16F4 strain (identified as FERM BP-2051), which had been incubated in a slant agar medium, was inoculated into 80 ml of a liquid culture medium comprising 1.0% starch and 3.0% soybean meal (pH 7.0 before sterilization) which was placed in a Sakaguchi's flask of 500 ml-capacity. The inoculated culture medium was incubated at 28°C for 3 days with rotatory shaking (135 rpm.) to provide a first seed culture. The first seed culture obtained was inoculated in 3 ml-portions into 80 ml-portions of the liquid culture medium having the same composition as above, which were separately placed in Sakaguchi's flasks. The inoculated culture media were incubated for 3 days under the same incubation conditions as above, to give the second seed culture. The resultant second seed culture (2 litres) was then inoculated to a culture medium (50 litres) of the same composition as above which had been sterilized at 120°C for 15 minutes and was placed in a tank-fermentor of 100 1-capacity. The so-inoculated culture medium was then cultured at 28°C for 2 days under aeration at a rate of 50 l of air per minute and under agitation at 200 rpm. to effect the submerged cultivation of the MH193-16F4 strain under aerobic conditions and obtain a third seed culture. The resultant third seed culture (12 litres) was inoculated into a productive culture medium (300 litres) comprising 2.0% of glycerin, 1.5% of soybean meal (available commercially under a tradename "Esusan Meat", a product of Ajinomoto Co. Ltd., Japan), 0.0025% of $K_2HPO_4$, 0.1125% of $KH_2PO_4$, 0.0005% of $CoCl_2 \cdot 6H_2O$, 0.03% of a silicone oil "KM72" (an antifoaming agent, a trade name of a product of Shinetsu Chemicals Co. Ltd., Japan) and 0.01% of a surfactant "Adekanol" (a trade name, product of Asahi Denka Kogyo Co. Ltd., Japan) which had preliminarily been sterilized at 125°C for 30 minutes and was placed in a tankfermentor of 570 1-capacity. The cultivation was conducted at 28°C for 7 days under agitation at 300 rpm. and under aeration at a rate of 150 l of air per minute for the first 24 hours of the cultivation and then at a rate of 300 l of air per minute after 24 hours of cultivation. After the completed cultivation, the culture broth obtained was mixed with diatomaceous earth as a filtration-aid and then filtered to give 250 l of the culture broth filtrate (pH 6.0).

Reference Example 2

The culture broth filtrate (250 l) obtained in the above Reference Example 1 was passed through a column of 15 l of a macroreticular adsorbent resin "Diaion HP-20" to effect the adsorption of the active substances by the adsorbent. After the adsorbent column was washed with 100 l of water and with 45 l of 50% aqueous methanol, the adsorbent column was eluted with 45 l of 70% aqueous methanol and then with 90 l of dry methanol, and the eluate was collected as the first fraction (53 l) and the second fraction (38 l) which both contained benanomicin A, as well as the third fraction (27 l) which contained benanomicin B, with the eluate being collected in fractions. The first fraction containing mostly benanomicin A was concentrated to 3 l under reduced pressure, followed by adjustment to pH 3.5 with dilute hydrochloric acid to deposit a precipitate of a red color.

The precipitate was collected by filtration and then dried in vacuo, whereby 152 g of a crude brown powder mainly comprising benanomicin A together with dexylosylbenanomicin A was obtained.

This crude powder (150 g) was dissolved in 600 ml of N,N-dimethylformamide. After saturation of the resultant solution with water vapor at room temperature for 3 days in a desiccator, a crystalline precipitate was deposited. The precipitate was collected by filtration and then dried under reduced pressure, thereby obtaining 29 g of benanomicin A-dimethylformamide solvate.

The filtrate, namely the mother liquor from which the crystalline precipitate had been removed by filtration was stored for use in recovery of dexylosylbenanomicin A therefrom as described in Example 2 given hereinafter.

The above-mentioned second fraction of the eluate was processed in the same way as the first fraction, thereby obtaining 14 g of benanomicin A-dimethylformamide solvate.

One gram of the benanomicin A-dimethylformamide solvate as obtained from said first fraction was dissolved in dimethyl sulfoxide (5 ml). The resultant solution was added dropwise under stirring into 300 ml of methanol, followed by stirring for 10 minutes to deposit a precipitate of a reddish brown color. The precipitate was filtered out and then dried under reduced pressure, to afford 935 mg of a purified benanomicin A as reddish brown powder.

The production of dexylosylbenanomicin A from benanomicin A is illustrated with reference to Example 1 below.

Example 1

Benanomicin A (1.05 g) was dissolved in 127 ml of an aqueous solution of 0.02 N sodium hydroxide, to which was then added 2.71 g of sodium methaperiodate (a product from Wako Pure Chemical Industries Ltd., Japan). The mixture obtained was stirred at room temperature for 40 minutes to effect oxidation of benanomicin A. Thereafter, the resulting reaciton solution was added with 0.8 ml of ethylene glycol and then stirred at room temperature for 15 minutes. The reaction solution was then passed through a column (800 ml) of a microporous non-ionic adsorbent resin, "Diaion HP-20", and this column was then washed with water (2 l) and subsequently eluted with 40% aqueous acetone to give an eluate containing the oxidation product. The eluate was collected in 100 ml-fractions. Fractions Nos. 7 to 12 of the eluate were combined and concentrated to a volume of about 20 ml, followed by adjustment to pH 2.5 by addition of 0.1 N hydrochloric acid to deposit a reddish brown precipitate. The precipitate was collected by centrifuging) (3000 rpm., 10 minutes) and then dried to afford 905 mg of the aforesaid diformyl compound (having an empirical formula $C_{38}H_{37}NO_{18}$) which was derived from benanomicin A by its periodate oxidation.

This diformyl derivative (200 mg) was dissolved in 25 ml of an aqueous solution of 0.01 N sodium hydroxide and the resulting aqueous solution was added with 15 mg of sodium borohydride (a product from Wako Pure Chemical Industries Ltd.) as a reducing agent. The mixture obtained was stirred at room temperature for 20 minutes to effect the reductive cleavage of said diformyl derivative. The resulting reaction solution containing dexylosylbenanomicin A as formed was adjusted to pH 3.0 by addition of 0.1 N hydrochloric acid and then stirred for 30 minutes and subsequently added with an aqueous solution of 0.1 N sodium hydroxide to adjust the pH to 7.5 and dissolve the deposited precipitate into the water phase. The resulting solution was passed through a column (100 ml) of "Diaion HP-20" and this column was then washed with water (200 ml) and eluted with 30% aqueous acetone for elution of the active substance from the column. The eluate was collected in 5 ml-fractions. Fraction Nos. 12 to 28 of the eluate were combined and concentrated to dryness to give 159 mg of a crude powder of reddish brown color. This crude powder was dissolved in 2 ml of dimethyl sulfoxide and the resulting solution was charged into a column (1 l) of a gel-filtration agent "Sephadex LH-20", which was then developed with methanol. The eluate was collected in 7 ml-fractions. The fractions Nos. 52 to 64 were combined and concentrated to a volume of about 5 ml. The concentrated solution was added with 0.01 N hydrochloric acid (5 ml) and a reddish brown precipitate deposited was then collected by centrifuging) (3000 rpm., 10 minutes). This precipitate was dried to afford 52.6 mg of dexylosylbenanomicin A as a pure product having a melting point of greater than 200°C.

Example 2 below illustrates the recovery and purification of dexylosylbenanomicin A from the culture broth obtained by cultivation of the benanomicinproducing MH193-16F4 strain.

Example 2

The filtrate (the mother liquor) which was obtained in the above Reference Example 2 and from which the crystalline precipitate of benanomicin A-dimethylformamide solvate had been collected by filtration was concentrated to dryness under reduced pressure to yield 130 g of a brownish crude powder. The crude powder

(5 g) was suspended in 200 ml of water. The resulting aqueous suspension was adjusted to pH 9.0 with 1 N aqueous sodium hydroxide to give a clear solution. The clear solution was re-adjusted to pH 2.5 with 1 N hydrochloric acid to afford 1.8 g of a brownish red precipitate. This precipitate was collected and the powder was dissolved in water (100 ml) and adjusted to pH 7.0 with 1 N aqueous sodium hydroxide, and the resulting solution was chromatographed by developing with 1% aqueous methanol on a reverse-phase silica gel column (1.0 l, Cosmosil $75C_{18}$-OPN) which had been pre-treated with 1% aqueous methanol. The eluate from the reverse-phase silica gel column was collected in fractions. The fractions containing a substance which was detected by HPLC [Precolumn: Cosmosil ${}_{10}C_{18}$, 4.6 mm in diameter x 50 mm in height, and Column: Cosmosil ${}_{5}C_{18}$, 4.6 mm in diameter x 150 mm in height (products of Nacalai Tesque, Inc., Japan); Mobile phase: 0.5% $KH_2PO_4$-MeOH (1 : 1), Flow rate: 1 ml/minute; Temperature: 40°C] at 22.7 minutes of retention time were combined and the combined solution was concentrated and then adjusted to pH 3.5 with 1N HCl to yield a brownish red powder (345 mg) of pure dexylosylbenanomicin A as the precipitate.

## Claims

1. An antifungal antibiotic, dexylosylbenanomicin A having the following formula (I)

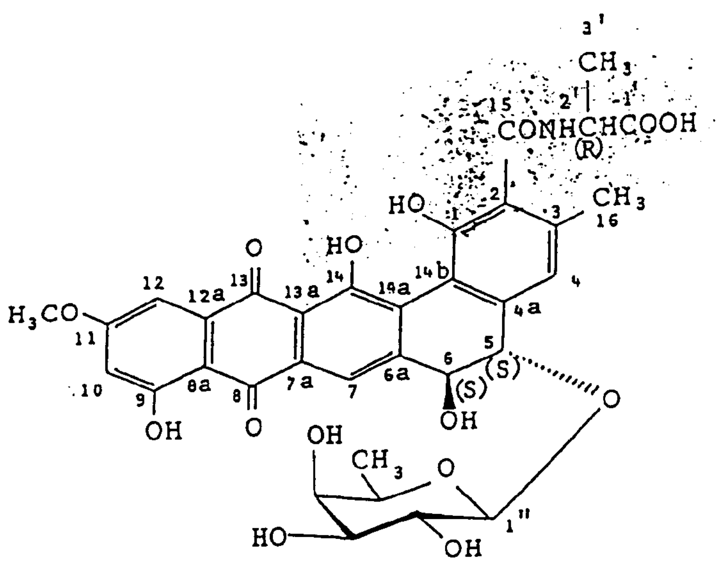

(I)

and salts thereof.

2. An antifungal composition for the therapeutic treatment of a fungal infection in mammalian animals, including man, which comprises an antifungally effective amount of dexylosylbenanomicin A having the formula (I) as defined in Claim 1 or a pharmaceutically acceptable salt thereof, as active ingredient, in association with a pharmaceutically acceptable solid or liquid carrier for the active ingredient.

3. A process for the production of dexylosylbenanomicin A from benanomicin A, which process comprises reacting benanomicin A with periodic acid or an alkali metal periodate in solution in water at room temperature, so that the adjacent hydroxyl groups of the xylosyl moiety of benanomicin A are oxidized to give a diformyl compound derived from benanomicin A that contains the two formyl groups and has an empirical formula $C_{38}H_{37}NO_{18}$, and then treating said diformyl compound with sodium borohydride or boron sodium cyanohydride to produce dexylosylbenanomicin A in the resulting reaction solution, and recovering dexylosylbenanomicin A from the reaction solution containing dexylosylbenanomicin A.

4. A process for producing simultaneously dexylosylbenanomicin A, benanomicin A and benanomicin B, which comprises cultivating the MH193-16F4 strain of actinomycetes (identified as FERM BP-2051) in a culture medium containing assimilable carbon and nitrogen sources under aerobic conditions until a substantial amount of dexylosylbenanomicin A is produced and accumulated in the culture along with benanomicins A and B, and then recovering dexylosylbenanomicin A, benanomicin A and benanomicin B separately or in mixture from the culture.

5. Use of dexylosylbenanomicin A or a pharmaceutically acceptable salt thereof for the manufacture of an antifungal agent.

## Patentansprüche

1. Antifungales Antibiotikum, Dexylosylbenanomicin A mit folgender Formel (I)

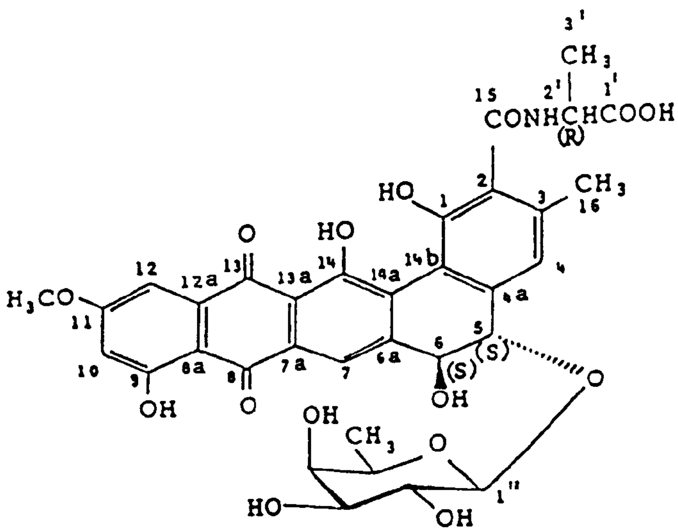

(I)

und Salze davon.

2. Antifungales Mittel für die therapeutische Behandlung einer Pilzinfektion bei Säugern, einschließlich Menschen, umfassend eine antifungal wirksame Menge von Dexylosylbenanomicin A mit der Formel (1) nach Anspruch 1 oder ein pharmazeutisch verträgliches Salz davon als Wirkstoff in Verbindung mit einem pharmazeutisch verträglichen festen oder flüssigen Träger für den Wirkstoff.

3. Verfahren zur Herstellung von Dexylosylbenanomicin A aus Benanomicin A, umfassend die Umsetzung von Benanomicin A mit Perjodsäure oder einem Alkalimetallperjodat in Lösung in Wasser bei Raumtemperatur, so daß die benachbarten Hydroxylgruppen der Xylosyleinheit von Benanomicin A oxidiert werden, wobei eine von Benanomicin A abgeleitete Diformylverbindung erhalten wird , die die zwei Formylgruppen enthält und die empirische Formel $C_{38}H_{37}NO_{18}$ aufweist, und anschließende Behandlung der Diformylverbindung mit Natriumborhydrid oder Bornatriumcyanohydrid, wobei Dexylosylbenanomicin A in der entstehenden Reaktionslösung hergestellt wird, und Gewinnung des Dexylosylbenanomicins A aus der Dexylosylbenanomicin A enthaltenden Reaktionslösung.

4. Verfahren zur gleichzeitigen Herstellung von Dexylosylbenanomicin A, Benanomicin A und Benanomicin B, umfassend die Züchtung des MH193-16F4 Stammes der Actinomyceten (identifiziert als FERM BP-2051) in einem Kulturmedium, das assimilierbare Kohlenstoff- und Stickstoffquellen enthält, unter aeroben Bedingungen, bis eine wesentliche Menge von Dexylosylbenanomicin A hergestellt und in der Kultur zusammen mit den Benanomicinen A und B angereichert ist, und anschließende Gewinnung von Dexylosylbenanomicin A, Benanomicin A und Benanomicin B getrennt oder im Gemisch aus der Kultur.

5. Verwendung von Dexylosylbenanomicin A oder eines pharmazeutisch verträglichen Salzes davon zur Herstellung eines antifungalen Mittels.

## Revendications

1. Un antibiotique antifongique, la dexylosylbénanomicine A ayant la formule suivante (I)

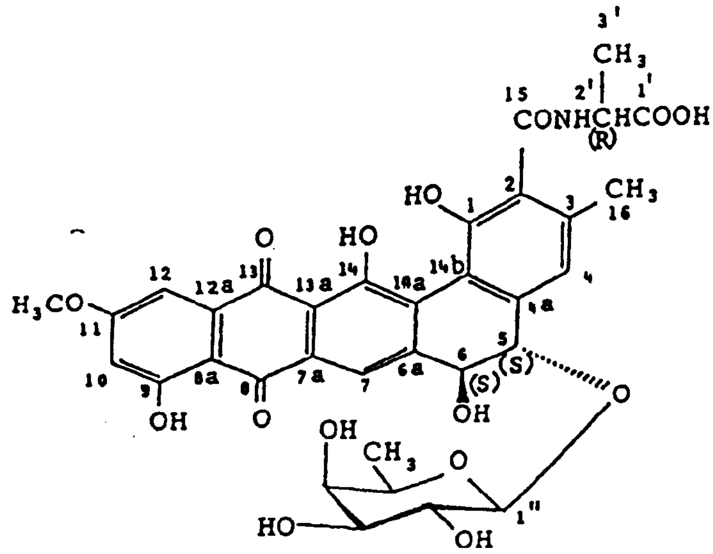

(I)

et les sels de celui-ci.

2. Une composition antifongique pour le traitement thérapeutique d'une infection fongique chez les animaux mammifères, y compris chez l'homme, qui comprend une quantité efficace du point de vue antifongique de dexylosylbénanomicine A ayant la formule (I) comme cela est défini dans la revendication 1 ou un sel de celle-ci acceptable du point de vue pharmaceutique, comme principe actif, en association avec un porteur solide ou liquide acceptable du point de vue pharmaceutique pour le principe actif.

3. Un procédé pour la production de la dexylosylbénanomicine A à partir de la bénanomicine A, dont le procédé comprend la réaction de la bénanomicine A avec l'acide périodique ou un périodate métallique alcalin en solution dans l'eau à la température ambiante, de sorte que les groupes hydroxyle adjacents de la moitié xylosyl de la bénanomicine A sont oxydés pour donner un composé diformyl dérivé de la bénanomicine A qui contient les deux groupes formyl et a une formule empirique $C_{38}H_{37}NO_{18}$, et ensuite le traitement dudit composé diformyl avec du borohydrure de sodium ou cyanohydrure de bore sodium pour produire la dexylosylbénanomicine A dans la solution de réaction résultante, et de récupération de la dexylosylbénanomicine A de la solution de réaction contenant la dexylosylbénanomicine A.

4. Un procédé pour produire simultanément la dexylosylbénanomicine A, la bénanomicine A et la bénanomicine B, qui comprend la culture de la souche d'actinomycètes MH193-16F4 (identifiée sous FERM BP-2051) dans un milieu de culture contenant des sources assimilables de carbone et d'azote dans des conditions d'aérobie jusqu'à ce qu'une quantité importante de dexylosylbénanomicine A soit produite et accumulée dans la culture avec les bénanomicines A et B, et ensuite de récupération de la dexylosylbénanomicine A, de la bénanomicine A et de la bénanomicine B séparément ou en mélange de la culture.

5. Utilisation de la dexylosylbénanomicine A ou un sel de celle-ci acceptable du point de vue pharmaceutique pour la fabrication d'un agent antifongique.

FIG.1

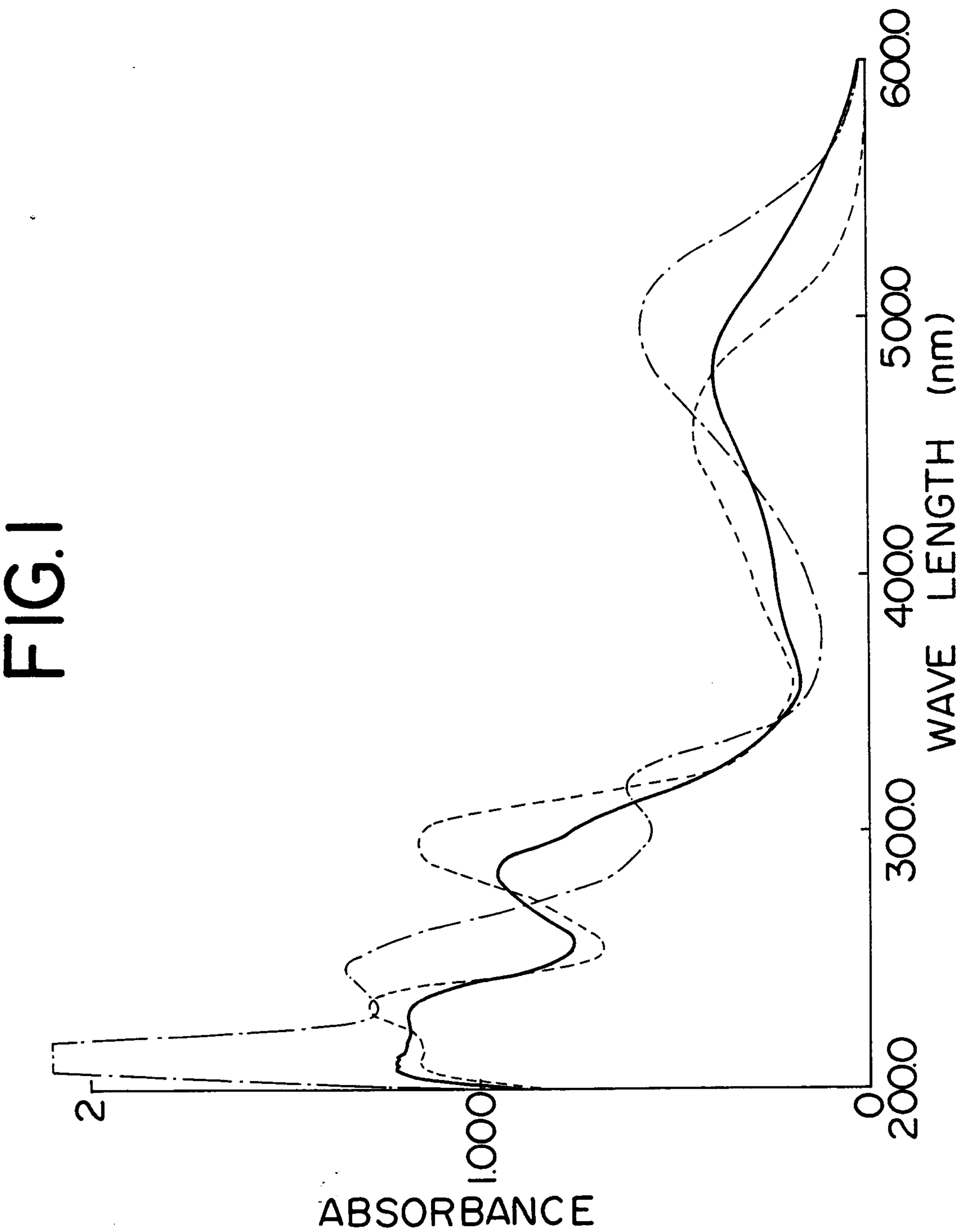

FIG. 2

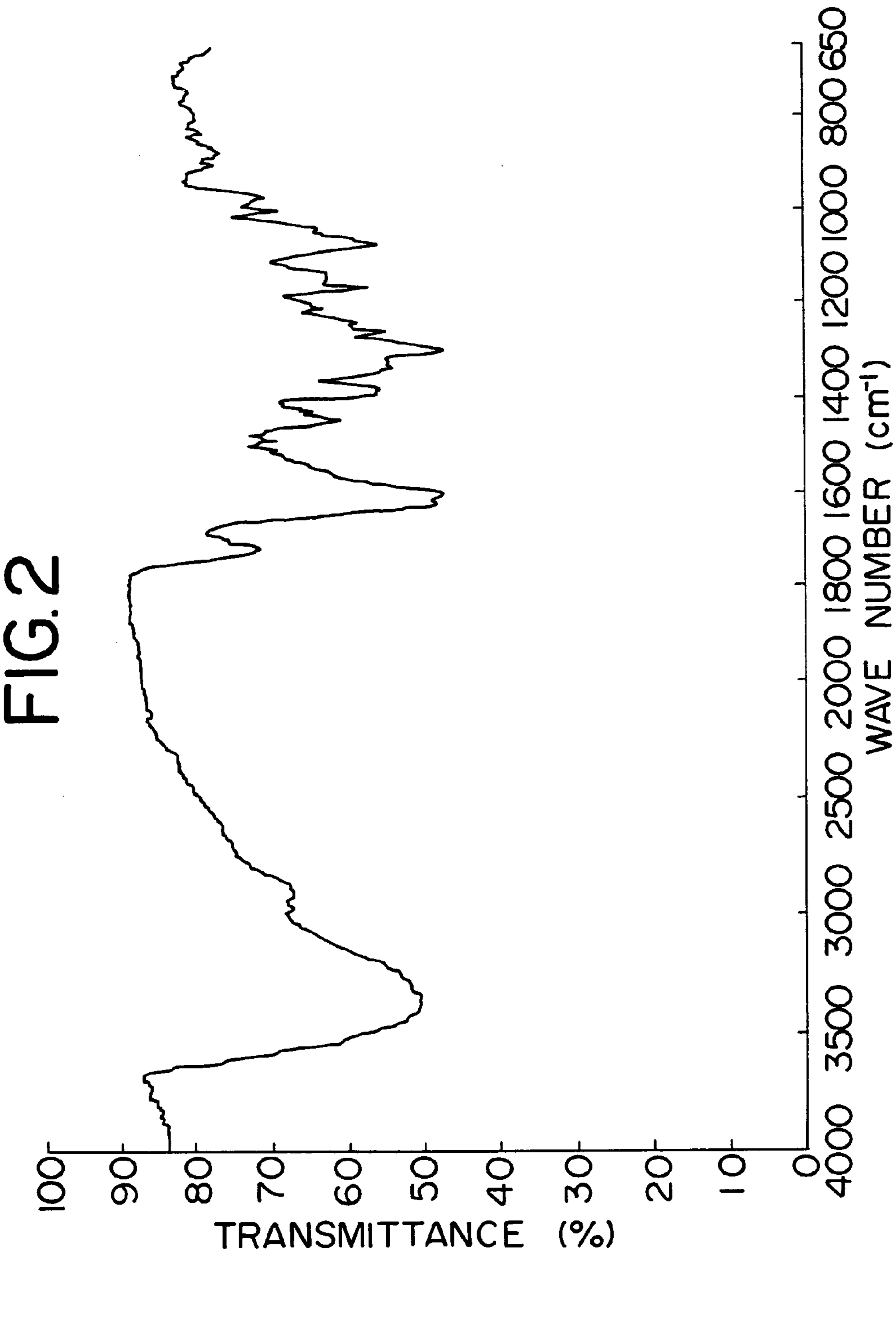
100
90
80
70
60
50
40
30
20
10
0
TRANSMITTANCE (%)
4000
3500
3000
2500
2000
1800
1600
1400
1200
1000
800
650
WAVE NUMBER (cm-1)

FIG. 3

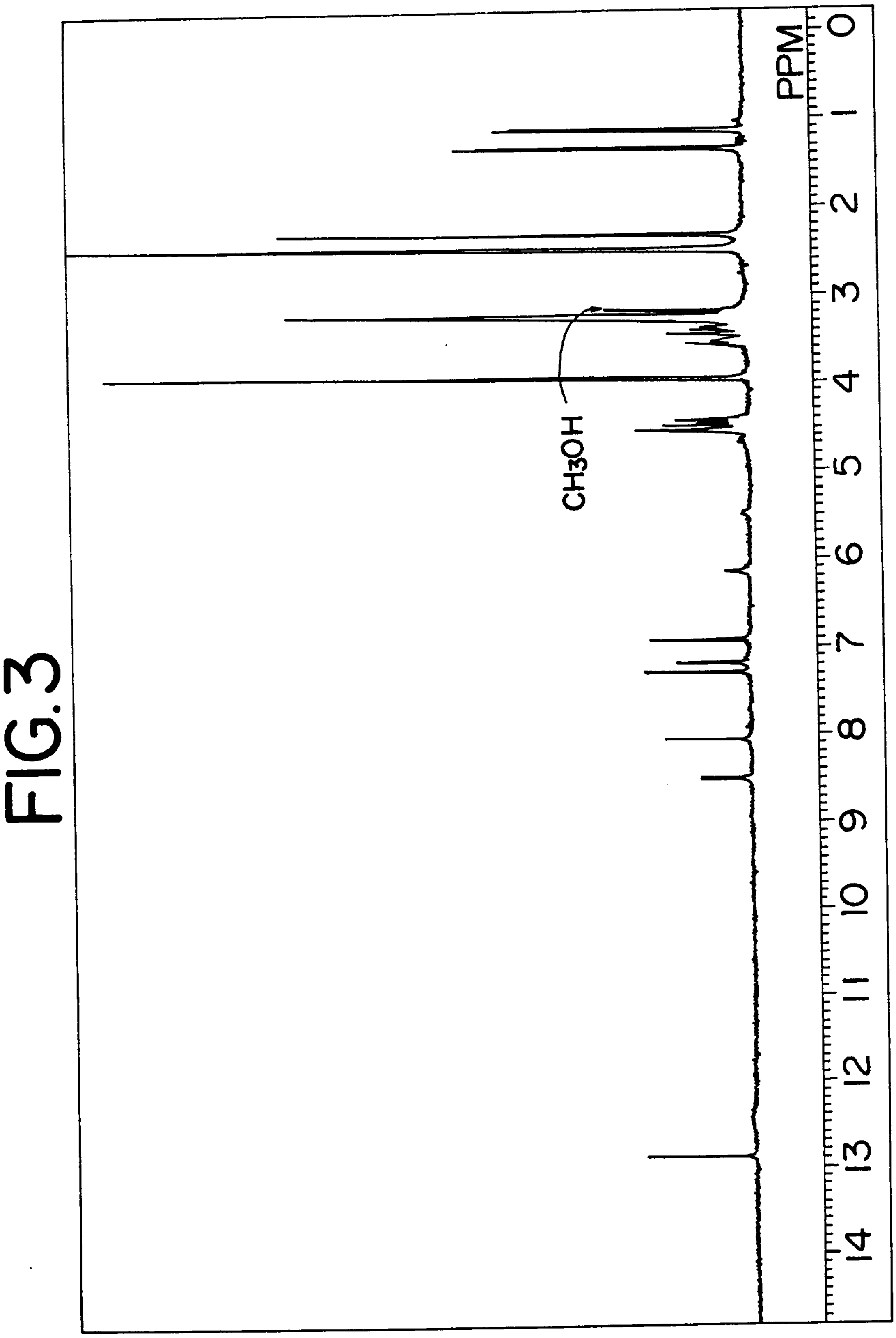

FIG.4

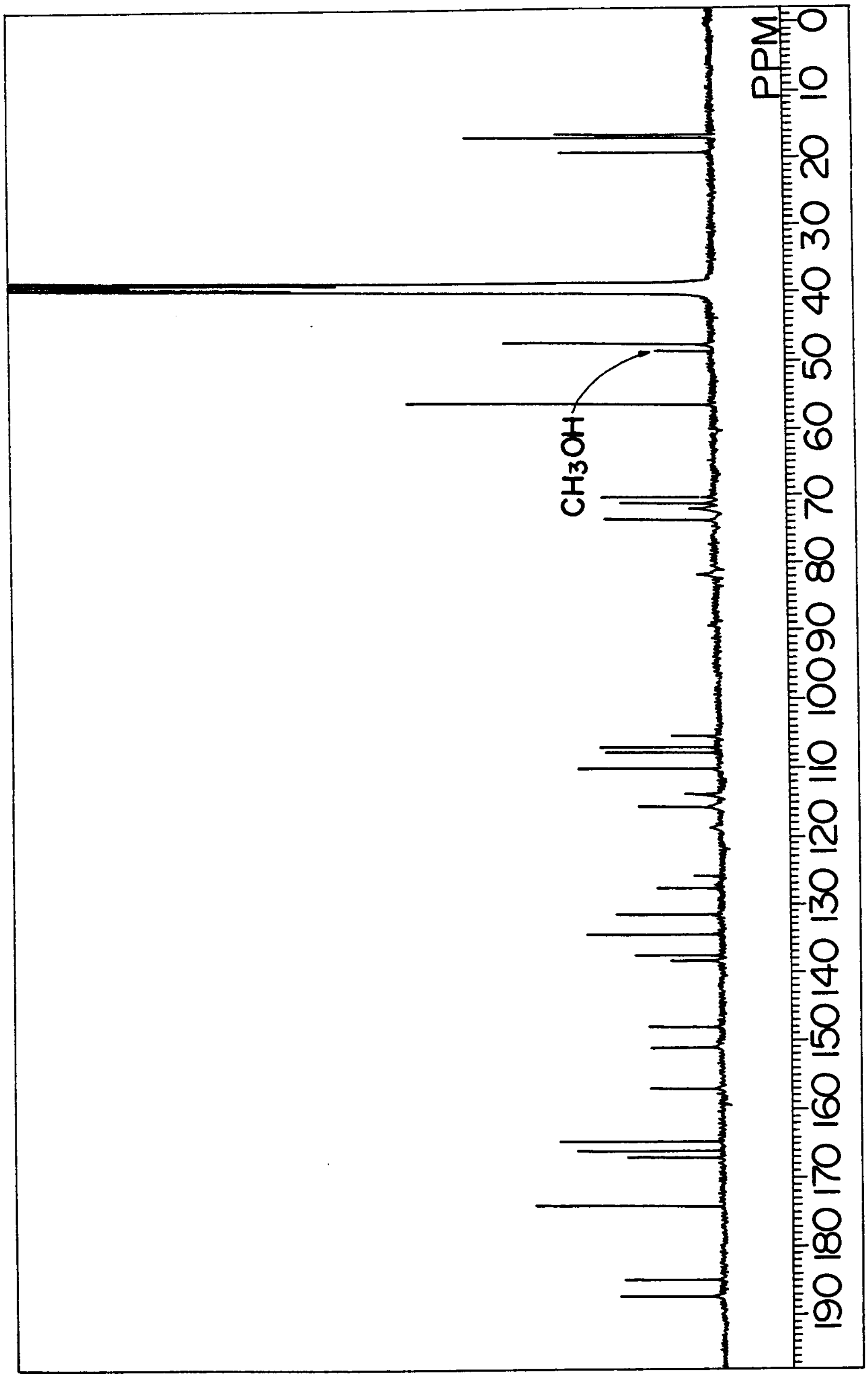
CH3OH
PPM
0
10
20
30
40
50
60
70
80
90
100
110
120
130
140
150
160
170
180
190